# EUROPÄISCHE PATENTANMELDUNG

(11) **EP 3 287 068 A1**
(43) Veröffentlichungstag der Anmeldung: **28.02.2018**
(21) Anmeldenummer: 17187155.1
(22) Anmeldetag: 22.08.2017
(51) Int. Cl.: A61B 5/00, A61B 5/03

(54) **ÜBERTRAGUNGSVORRICHTUNG ZUR ÜBERTRAGUNG VON HIRNPARAMETER-SENSORDATEN**

(30) Priorität: 24.08.2016 DE 202016005183 U
(71) Anmelder: Raumedic AG, 95213 Münchberg (DE)
(72) Erfinder: Göhler, Karlheinz, 08297 Zwönitz (DE); Peitsch, Peter, 99099 Erfurt (DE); Jurisch, Reinhard, 99438 Meckfeld (DE)
(74) Vertreter: Rau, Schneck & Hübner Patentanwälte Rechtsanwälte PartGmbB

(57) **Zusammenfassung**

Eine Übertragungsvorrichtung zur Übertragung von Hirnparameter-Sensordaten umfasst
- eine Empfangseinheit mit einer Antenne, die mit einer Sendeeinheit eines Hirnparametersensors zur Datenübertragung drahtlos verbindbar ist,
- eine Datenverarbeitungseinrichtung (4), die mit der Empfangseinheit in Signalverbindung steht,
- eine Hochfrequenzquelle (14) zur Erzeugung einer Datenübertragungs-Trägerfrequenz,
- wobei die Hochfrequenzquelle (14) in ein Gehäuse (6) der Datenverarbeitungseinrichtung (4) integriert ist.

Es resultiert eine Übertragungsvorrichtung, die für den Einsatz in einer häuslichen Patentienumgebung ausgelegt ist.

## Beschreibung

Die vorliegende Patentanmeldung nimmt die Priorität der deutschen Gebrauchsmusteranmeldung 20 2016 005 183.1 in Anspruch, deren Inhalt durch Bezugnahme hierin aufgenommen wird.

Die Erfindung betrifft eine Übertragungsvorrichtung zur Übertragung von Hirnparametern-Sensordaten.

Derartige Übertragungsvorrichtungen sind bekannt aus der WO 2009/043 431 A1 und WO 2013/017 440 A1. Die US 2009/0143696 A1 beschreibt eine Übertragungsvorrichtung für Hirnparameterdaten mit einem Implantat sowie einer externen Empfangs- und Datenverarbeitungseinheit.

Es ist eine Aufgabe der vorliegenden Erfindung, eine Übertragungsvorrichtung der eingangs genannten Art derart weiterzubilden, dass sie für den Einsatz in einer häuslichen Patientenumgebung ausgelegt ist.

Diese Aufgabe ist erfindungsgemäß gelöst durch eine Übertragungsvorrichtung mit den im Anspruch 1 angegebenen Merkmalen.

Erfindungsgemäß wurde erkannt, dass es nicht zwingend erforderlich ist, eine Hochfrequenzquelle, die für die Übertragung der Hirnparameter-Sensordaten vom in der Regel implantierten Hirnparametersensor hin zur Empfangseinheit erforderlich ist, nicht als in Bezug auf die Datenverarbeitungseinrichtung externe geschirmte Komponente auszuführen. Soweit geeignete Vorkehrungen zur Abschirmung getroffen werden, ist es durchaus möglich, die Hochfrequenzquelle in das Gehäuse der Datenverarbeitungseinrichtung zu integrieren, ohne dass dies zu unerwünschten Störungen führt. Es resultiert eine sehr kompakte Übertragungsvorrichtung, die bequem beispielsweise wie ein Mobiltelefon am Körper getragen werden kann. Das Gehäuse weist eine metallische Frontplatte und eine metallische Basisplatte auf, die beide von einem umlaufenden Gehäuserahmen aus Kunststoff getragen werden. Eine derartige Gehäuseausführung ist leicht und kompakt. Es hat sich herausgestellt, dass es zur ausreichenden Abschirmung der Hochfrequenzquelle nicht erforderlich ist, den umlaufenden Gehäuserahmen ebenfalls aus Metall auszuführen.

Eine Komponentenanordnung nach Anspruch 2 erleichtert einen kompakten Aufbau der Datenverarbeitungseinrichtung weiter. Aufwendige Verkabelungen können entfallen. Signalverbindungen zwischen den Elektronik-komponenten und/oder Komponenten der Hochfrequenzquelle können als Leiterbahnen auf der Leiterplatte ausgeführt sein.

Eine Abschirmeinheit nach Anspruch 3 vermeidet sicher unerwünschte Störungen durch die Hochfrequenzquelle.

Eine Gehäuseausführung nach Anspruch 4 lässt sich einfach montieren. Die Frontplatte kann bei einem derartigen Gehäuseaufbau gleichzeitig die Leiterplatte darstellen.

Eine Schnittstelle nach Anspruch 5 ermöglicht eine Weiterleitung und externe Auswertung der Sensordaten. Über die Schnittstelle kann ein mobiler Speicher angekoppelt werden und/oder es können über die Schnittstelle die Sensordaten kabelgebunden oder drahtlos übertragen werden. Bei der Schnittstelle kann es sich um eine USB-Buchse handeln. Auch eine WLAN-Schnittstelle ist möglich.

Die Vorteile eines Sensorsystems nach Anspruch 6 entsprechen denen, die vorstehend unter Bezugnahme auf die erfindungsgemäß im Übertragungsbericht bereits erläutert wurden. Der Hirnparametersensor kann so ausgeführt sein wie beispielsweise in der WO 2009/043 431 A1 und der WO 2013/017 440 A1 beschrieben.

Ein Ausführungsbeispiel der Erfindung wird nachfolgend anhand der Zeichnung näher erläutert. In dieser zeigen:
- Fig. 1: eine Übertragungsvorrichtung zur Übertragung von Hirnparametern-Sensordaten als Bestandteil eines Sensorsystems zur Messung, Übertragung, Verarbeitung und Darstellung eines Hirnparameters; und
- Fig. 2: einen schematischen Schnitt durch eine Datenverarbeitungseinrichtung der Übertragungsvorrichtung, aufweisend eine Hochfrequenzquelle, die in ein Gehäuse der Datenverarbeitungseinrichtung integriert ist.

Eine in der Figur insgesamt dargestellte Übertragungsvorrichtung 1 ist Bestandteil eines Sensorsystems zur Messung, Übertragung, Verarbeitung und Darstellung eines Hirnparameters. Nicht dargestellte weitere Komponenten des Sensorsystems sind ein Hirnparametersensor sowie ein externes Auswertesystem. Die Übertragungsvorrichtung 1 ist zur telemetrischen Hirnparameter-Messung und insbesondere zur Hirndruckmessung in einer häuslichen Patientenumgebung ausgelegt.

Die Übertragungsvorrichtung 1 umfasst eine Empfangseinheit 2 mit einer Antenne 3, die mit einer Sendeeinheit des nicht dargestellten Hirnparametersensors in drahtloser Signalverbindung steht. Als Hirnparametersensor kann ein implantierbarer Hirndrucksensor zum Einsatz kommen, wie er beispielsweise bekannt ist aus der WO 2009/043 431 A1 und aus der WO 2013/017 440 A1.

Bei der Antenne 3 handelt es sich um eine Ringantenne, die als Spule ausgeführt ist und die als Schwingkreis geschaltet sein kann.

Zur Übertragungsvorrichtung 1 gehört weiterhin eine Datenverarbeitungseinrichtung 4, die mit der Empfangseinheit 2 über ein Antennenkabel 5 in Signalverbindung steht. Das Antennenkabel 5 hat eine Länge im Bereich zwischen 1 m und 1,5 m. Die Datenverarbeitungseinrichtung 4 hat ein Gehäuse 6, welches in etwa die Abmessungen eines typischen Smartphones hat. Die Datenverarbeitungseinrichtung 4 kann in einer Gürteltasche am Körper des Patienten getragen werden.

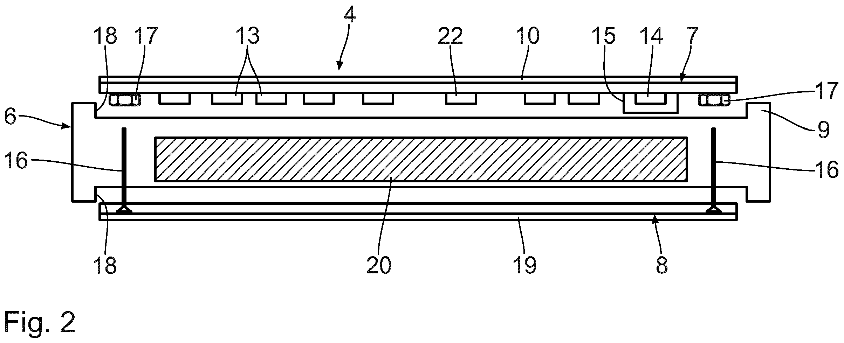

Figur 2 zeigt Details zum Aufbau der Datenverarbeitungseinrichtung 4. Das Gehäuse 6 der Datenverarbeitungseinrichtung 4 umfasst eine metallische Frontplatte 7, eine metallische Basisplatte 8 und einen umlaufenden Gehäuserahmen 9 aus Kunststoff, z. B. aus Polyoxymethylen (POM).

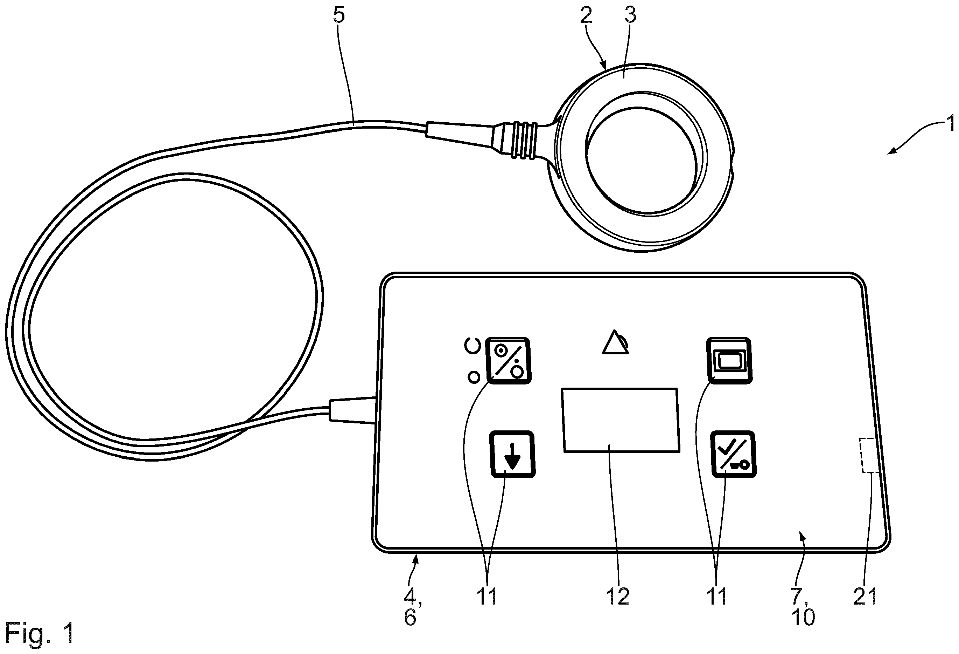

Die Frontplatte 7 stellt gleichzeitig eine Leiterplatte dar, auf deren Rückseite sämtliche Elektronikkomponenten der Datenverarbeitungseinrichtung 4 angeordnet sind. Eine Frontseite der Frontplatte 7 trägt eine Folientastatur 10 mit insgesamt vier Bedientasten 11 und einem zentralen Display 12 (vgl. Fig. 1).

Rückseitig trägt die Frontplatte/Leiterplatte 7 die Elektronikkomponenten insbesondere in Form einer SMD-Bestückung mit integrierten Logik- und Speicherchips 13. Rückseitig trägt die Leiterplatte 7 auch eine Hochfrequenzquelle 14. Letztere dient zur Erzeugung einer Datenübertragungs-Trägerfrequenz zur Übertragung von Sensor-Rohdaten vom Hirnparametersensor über die Empfangseinheit 2 zur Datenverarbeitungseinrichtung 4. Die Hochfrequenzquelle 14 ist in das Gehäuse 6 der Datenverarbeitungseinrichtung 4 integriert.

Die Datenverarbeitungseinrichtung 4 kann die Funktion eines RFID-Readers haben.

Zum Abschirmen der Hochfrequenzquelle 14 gegenüber den Elektronik-komponenten 13 der Datenverarbeitungseinrichtung 4 dient eine metallische Abschirmeinheit 15. Letztere ist als die Hochfrequenzquelle 14 umgebendes Blechgehäuse ausgeführt.

Mithilfe von Schrauben 16, die durch Löcher in der Basisplatte 8 gesteckt sind, und von fest mit der Frontplatte 7 verbundenen Gewindemuttern 17 werden die Frontplatte 7 und die Basisplatte 8 miteinander verschraubt, wobei die Platten 7, 8 in Ausnehmungen 18 im Gehäuse-Rahmen 9 einrücken, sodass die Frontplatte 7 einerseits und die Basisplatte 8 andererseits bündig in den umlaufenden Gehäuse-Rahmen 9 im montierten Zustand eingesetzt sind.

Auch die Basisplatte 8 trägt zur Sichtseite hin eine Folie 19. Die Folie 19 kann ein Typenschild der Datenverarbeitungseinrichtung 4 bzw. des gesamten Sensorsystems 1 aufweisen. Die Folie 19 kann weiterhin insbesondere Dekorationszwecken dienen und beispielsweise Befestigungselemente abdecken, mit denen die Basisplatte 8 und der Gehäuse-Rahmen 9 sowie die Frontplatte 7 miteinander verbunden sind.

Im Gehäuse 6 ist zusätzlich noch ein Akkumulator 20 zur Stromversorgung der Übertragungsvorrichtung 1 untergebracht. Über ein an die Datenverarbeitungseinrichtung 4 anschließbares Netzteil (nicht dargestellt) kann der Akkumulator 20 geladen werden.

Die Datenverarbeitungseinrichtung 4 hat weiterhin noch eine Schnittstelle 21, die in der Fig. 1 schematisch gestrichelt angedeutet ist und die als USB-Buchse ausgeführt sein kann. Hier kann über einen USB-Stick eine Übertragung von Hirnparameterdaten von einem Speicher 22 der Datenverarbeitungseinrichtung 4 auf ein externes Gerät erfolgen. Alternativ oder zusätzlich kann die Schnittstelle 21 zur drahtlosen Signalübertragung, beispielsweise als WLAN-Schnittstelle, ausgeführt sein.

Über die Schnittstelle 21 können die Hirnparameterdaten zum externen Auswertesystem übertragen werden. Dieses externe Auswertesystem kann an einem Klinikstandort stationiert sein.

Mit Hilfe der Elektronikkomponenten 13 der Datenverarbeitungseinrichtung 4 kann eine Berechnung gemessener Hirnparameterwerte stattfinden.

Zwischen dem Hirnparametersensor und der Empfangseinheit 2 findet eine verschlüsselte Übertragung der vom Sensor gemessenen Hirnparameter-Rohdaten statt. In der Datenverarbeitungseinrichtung 4 erfolgt mit Hilfe der Elektronikkomponenten 13 eine Dekodierung dieser verschlüsselten Daten insbesondere in ein gängiges Datenformat, z. B. in ein .csv-Format, statt.

Ein so aufbereiteter Hirnparameter-Datensatz umfasst einen Header, in dem neben den eigentlichen Hirnparameterdaten noch zusätzliche Informationen stehen, insbesondere Identifikationsdaten zur Identifikation des Patienten, Start- und Stoppzeiten der Hirnparametermessung sowie gegebenenfalls auch weitere Daten, die der Patient selbst über die Folientatstatur 10 eingeben kann.

Die gesamte Übertragungsvorrichtung 1 ist wetterfest und insbesondere wasserdicht ausgeführt. Der Akkumulator 20 ist so ausgelegt, dass ohne Netzteil-Einsatz ein mobiler Betrieb der Übertragungsvorrichtung 1 über mehr als einen Tag möglich ist. Entsprechend ist auch der Speicher 22 dimensioniert, dessen Kapazität zur Sicherung einer Datenmenge ausreicht, die sogar während mehrerer Monate erfasst werden können.

## Patentansprüche

1. Übertragungsvorrichtung (1) zur Übertragung von Hirnparameter-Sensordaten,
- mit einer Empfangseinheit (2) mit einer Antenne (3), die mit einer Sendeeinheit eines Hirnparametersensors zur Datenübertragung drahtlos verbindbar ist,
- mit einer Datenverarbeitungseinrichtung (4), die mit der Empfangseinheit (2) in Signalverbindung steht,
- mit einer Hochfrequenzquelle (14) zur Erzeugung einer Datenübertragungs-Trägerfrequenz,
- wobei die Hochfrequenzquelle (14) in ein Gehäuse (6) der Datenverarbeitungseinrichtung (4) integriert ist,
- wobei das Gehäuse (6) eine metallische Frontplatte (7) und eine metallische Basisplatte (8) aufweist, die beide von einem umlaufenden Gehäuserahmen (9) aus Kunststoff getragen werden.

2. Übertragungsvorrichtung nach Anspruch 1, **dadurch gekennzeichnet, dass** Elektronikkomponenten (13) der Datenverarbeitungseinrichtung (4) auf einer einzigen Leiterplatte (7) im Gehäuse (6) angeordnet sind, wobei auch die Hochfrequenzquelle (14) auf der Leiterplatte (7) angeordnet ist.

3. Übertragungsvorrichtung nach Anspruch 1 oder 2, **gekennzeichnet durch** eine metallische Abschirmeinheit (15) zum Abschirmen der Hochfrequenzquelle (14) gegenüber den Elektronik-Komponenten (13) der Datenverarbeitungseinrichtung (4).

4. Übertragungsvorrichtung nach einem der Ansprüche 1 bis 3, **dadurch gekennzeichnet, dass** das Gehäuse (6) aus exakt drei Komponenten besteht, nämlich der Frontplatte (7), der Basisplatte (8) und dem Gehäuserahmen (9).

5. Übertragungsvorrichtung nach einem der Ansprüche 1 bis 4, **gekennzeichnet** für eine Schnittstelle (21) zum Übertragen von Sensordaten an ein externes Gerät.

6. Sensorsystem mit einer Übertragungsvorrichtung (1) nach einem der Ansprüche 1 bis 5 und mit einem Hirnparametersensor.
